# EUROPEAN PATENT SPECIFICATION

(11) **EP 2 489 269 B1**
(45) Date of publication and mention of the grant of the patent: **12.10.2016**
(21) Application number: 11155396.2
(22) Date of filing: 22.02.2011
(51) Int. Cl.: A01N 59/16, A01N 25/08, A01K 1/015, A01P 1/00

(54) **Composition comprising a biocidal composite**
Zusammensetzung mit einem bioziden Verbundstoff
Composition comportant un composite biocide

(30) Priority: 21.02.2011 GR 2011100101
(43) Date of publication of application: 22.08.2012
(73) Proprietor: Geohellas S.A., 175 64 Palaio Faliro (GR)
(72) Inventor: KACANDES, George, Athens 17564 (GR); DROSSOS, Emmanuel, 175 64 Palaio Faliro (GR)
(74) Representative: D Young & Co LLP

(56) References cited:
- EP-A2- 0 109 276
- EP-A2- 0 251 783
- WO-A1-00/00690
- WO-A1-2007/051996
- WO-A2-99/33335
- FR-A1- 2 793 386
- US-B1- 6 287 550
- MENESI J ET AL: "Photocatalysis on silver-layer silicate/titanium dioxide composite thin films at solid/vapour interface", CATALYSIS TODAY, ELSEVIER, NL, vol. 144, no. 1-2, 15 June 2009 (2009-06-15), pages 160-165, XP026096241, ISSN: 0920-5861, DOI: 10.1016/J.CATTOD.2009.02.030 [retrieved on 2009-04-29]
- JUDIT MÉNESI ET AL: "The Effect of Transition Metal Doping on the Photooxidation Process of Titania-Clay Composites", INTERNATIONAL JOURNAL OF PHOTOENERGY, vol. 2008, 1 January 2008 (2008-01-01), pages 1-9, XP55025376, ISSN: 1110-662X, DOI: 10.1155/2008/846304
- K. D. BRUNT ET AL: "A Group of More Friendly Biocides", SURFACE COATINGS INTERNATIONAL PART B: COATINGS TRANSACTIONS, 30 November 1997 (1997-11-30), pages 473-475, XP55025363, DOI: 10.1007/BF02692718
- ANONYMOUS: 'JMAC Silver Biocide', [Online] 24 July 2008, XP055071741 Retrieved from the Internet: <URL:http://www.chemlink.co.uk/jmacbrochure 1107.pdf> [retrieved on 2013-07-17] & [Online] 17 July 2013, XP055071764 Retrieved from the Internet: <URL:http://web.archive.org/web/*/http://ww w.chemlink.co.uk/jmacbrochure1107.pdf> [retrieved on 2013-07-17]

## Description

### TECHNICAL FIELD

The present invention relates to a composition suitable for use in animal litter products and animal bedding products. The invention also relates to methods of preparing the same, and the use of transition metal ion/carrier composites for reducing bacterial growth and/or odours caused by animal urine and faeces in animal litter/bedding.

### BACKGROUND OF THE INVENTION

Animal litters are often employed in domestic situations where animals, particularly cats, urinate and defecate in a litter tray containing an animal litter. Alternatively, animal litters can be used as bedding materials for domestic or non-domestic animals. However, one problem that arises when using animal litters is the resulting build up of bacteria and odours which are caused by the presence of urine and faeces.

The presence of bacteria and bacterial growth in animal litter and bedding areas are major health concerns because bacteria can cause sickness and fatal disease in both animals and humans. Bacteria also reduce productivity in farm animals. Areas where such concerns exist include for example animal pens, stables, coops, and litter trays in homes. Other problematic organisms that can flourish in these areas include moulds, fungi, and algae. The problems associated with the growth of bacteria and other problematic organisms in litter and bedding materials are compounded by the fact that these areas are often wet or damp.

One specific problem caused by the build up of bacteria and bacterial growth is that it leads to the development of problematic malodours. In particular, ammonia malodours can be generated by the breakdown of urine by bacterial enzymes to produce urea and ammonia gas. At low concentrations, ammonia is an undesirable nuisance odour, but if ammonia is allowed to accumulate in a confined area it can be toxic to animals and humans. In these circumstances, ammonia production is a function of bacterial growth. The most direct way therefore to decrease ammonia levels in animal quarters is to inhibit bacterial growth.

Animal litters typically contain an absorbent material such as clay. Generally, animal litters, more specifically cat litters, come in two different kinds: traditional absorbent litter and clumping litter. Traditional litter absorbs the liquid, typically distributing the liquid throughout the absorbent material. In contrast, clumping litter, as the name suggests, clumps together when wet and forms a solid mass distinct from any unaffected litter. The used litter can then be more easily separated from the litter tray leaving behind unused litter for continued use.

Previous attempts to address the development of urine odours in animal litter boxes have employed known urease inhibitors. For example, EP 0 109 276 A describes the use of soluble salts of transition metal elements in animal litter compositions. Such compositions are prepared by slurrying an absorbent material in a solution of said transition metal salt, followed by drying.

Other attempts, e.g. US 6,287,550, WO 99/33335, have involved the use of transition metal ions in order to inhibit the formation of odours. In particular, clay cat litter is sprayed with an aqueous solution containing a dissolved transition metal compound such as zinc chloride.

FR 2793 386 describes a porous carrier combined with a transition metal-exchanged zeolite as a litter for domestic animals.

Other attempts, e.g. US 4,494,482, have involved the use of organic antibacterial active agents such as halogenated aromatic hydrocarbon bacteriostats.

More recently, e.g. WO 2006/086410 and US 2009/0314215, attempts have involved the use of additives which provide release of fragrance or odour masking scent only when the litter is used by a cat or other animal.

There remains a need to provide alternative and improved compositions for use in reducing malodours in animal litter/bedding products in order to address the problems associated with bacterial growth in environments in which animals are kept.

### SUMMARY OF THE INVENTION

In one aspect, the invention provides an animal litter product comprising a composition comprising (i) an absorbent material which is a clay and (ii) a biocidal composite which comprises an anti-bacterial transition metal compound and a carrier, wherein the transition metal compound is supported on the carrier material; and wherein the carrier material is titanium dioxide; and wherein the transition metal compound is present in the composition in an amount of less than 0.60ppm; and wherein the transition metal compound is silver chloride.

The present invention also provides a process for preparing a composition as defined in any one of claims 1-2, comprising applying a liquid suspension of said biocidal composite to said absorbent material. Preferably, the biocidal composite solution is applied by spraying.

In a further aspect, the invention relates to the use of a biocidal composite as defined in any one of claims 1-2 to reduce bacteria in an absorbent material, wherein the absorbent material is part of an animal litter product.

The composition of the invention effectively prevents bacterial growth and reduces the level of bacteria over an extended period of time. As well as addressing the direct health problems caused by the presence of bacteria in animal litters and/or animal bedding, the compositions of the invention also reduce the development of malodours caused by the presence of bacteria.

### DETAILED DESCRIPTION OF THE INVENTION

The composition of the invention may be used as animal litter.

### Absorbent Material

The absorbent material is suitable for use in an animal litter product. As such, the absorbent material is solid and is capable of absorbing liquid, e.g. urine. The absorbent material is a clay.

Clays generally absorb large quantities of liquid and are safe and non-irritating to animals. Suitable clays include, sepiolite, zeolites, saponite, tobermite, marls, attapulgite, bentonite, kaolinite (such as Georgia White clay), halloysite, montmorillonite, smectite, vermiculite, hectorite, diatomaceous earth (diatomite), Fuller's earth, synthetic cement, other similar materials, and mixtures thereof. In addition, calcined preparations of the above-mentioned clays may be employed.

Preferred clays include attapulgite, sepiolite, bentonite, montomorillonite, saponite, and diatomite. Most preferred are attapulgite, saponite or mixtures thereof.

The absorbent material employed in the present invention should be suitable for use as an animal litter. In this context, both traditional absorbent clay animal litters and clumping animal litters can be used in the present invention.

### Biocidal Composite

The biocidal composite employed in the composition of the present invention comprises an anti-bacterial transition metal compound and a carrier. The anti-bacterial transition metal compound is silver chloride and the carrier is titanium dioxide.

The transition metal compound (silver chloride) is supported on the (titanium dioxide) carrier material. For example, the transition metal compound may be supported within the carrier material thus the transition metal compound is incorporated in a protective sponge provided by the carrier material. Alternatively or additionally, the transition metal compound may be supported on the surface of the carrier material. Biocidal composites employed in the present invention in which a transition metal compound is supported on a carrier material have been observed to provide unexpected improved anti-bacterial properties. The carrier material is believed to provide an enlarged surface allowing the transition metal compound to disperse, preferably disperse evenly, over the surface of the carrier material and/or within the carrier material. Such a dispersion appears to reduce aggregation of the transition metal compound in the composite, and therefore maximise the anti-bacterial effect of the transition metal compound.

For example, a biocidal composite useful in the present invention comprises a transition metal compound deposited on a carrier wherein the carrier is in particulate form.

The transition metal compound is silver chloride which exhibits antibacterial properties.

The transition metal compound employed in the present invention is preferably incorporated into the biocidal composite in the form of sub-micronparticles.

The silver chloride may be present at a level of from 1-75% by weight of the carrier, preferably 10-60% by weight, more preferably 15-25% by weight, even more preferably 20% by weight. The composite comprises silver chloride deposited on a titanium dioxide carrier at the above levels.

As described above, the purpose of the titanium dioxide carrier material employed in the biocidal composite is to provide support for an anti-bacterial silver metal. The carrier may also aid slow and controlled release of the antibacterial silver metal.

Titanium dioxide is used as the carrier and provides improved performance.

In one embodiment, the surface area of the carrier should be extended. That is, the surface area of the carrier should be significantly greater than the nominal geometric
surface area. The carrier employed in the composition of the invention is preferably about 1-3 microns in diameter.

Such composites can be prepared by forming a slurry of the titanium dioxide carrier material in an aqueous solution of a salt or soluble compound of a silver and reacting with a compound containing the chloride anion of the desired silver chloride antimicrobial compound. For example, titanium dioxide may be slurried in an aqueous solution of silver nitrate and reacted with sodium chloride to precipitate silver chloride on the titanium dioxide.

Biocidal composites suitable for use in the present invention are described in EP0251783A, US 6,444,726, and Corbett, R.J., International Journal of Cosmetic Science 18, 151-165 (1996).

The biocidal composite comprises silver and titanium dioxide, i.e. a silver/titanium dioxide composite. The silver is included in the composite in the form of silver ions. The silver ions are incorporated using silver chloride. Silver chloride/titanium dioxide composites suitable for use in the present invention include, but are not limited to, commercially available composites sold in the UK under the trade name BIOMASTER by the company ADDMASTER. In particular, BIOMASTER TD100 and BIOMASTER AT100 are suitable for use as the biocidal composite in the present invention.

The effectiveness of the composite employed in the present invention is such that the transition metal compound can be applied to the absorbent material at remarkably low concentrations. The low concentrations made possible by the present invention still provide an effective reduction in bacterial growth. In fact, the low concentrations
made possible by the present invention still provide reductions in bacterial growth just as good as or better than commercially available animal litters. Surprisingly, it is possible to achieve effective anti-bacterial activity using a biocidal composite as described in any embodiment above in an amount from about 0.001 to about 2% by weight based on the weight of the absorbent material. More preferably, an amount of biocidal composite is used in an amount ranging from about 0.01 to about 1%, even more preferably from about 0.01 to about 0.5%, by weight based on the weight of the absorbent material.

For example, biocidal composites can be employed at low dosages less than about 3 parts per million (ppm), preferably less than about 2.5 ppm based on the weight of the composition product, e.g. the clay product. Accordingly, at low dosages the amount of biocidal composite can range from about 1.8 to about 2.5 ppm based on the weight of the composition product, e.g. the clay product. More preferably, the amount of biocidal composite can range from about 2.0 to about 2.2 ppm based on the weight of the composition product, e.g. the clay product.

The transition metal compound (silver chloride) is employed in the composition product, e.g. the clay product, at levels less than about 0.60 ppm, preferably less than about 0.50 ppm, based on the weight of the composition product, e.g. the clay product. In particular, the amount of transition metal compound (e.g. silver chloride) in the product is from about 0.40 to about 0.50 ppm based on the weight of the composition product, e.g. the clay product. This correlates to an amount of transition metal (e.g. silver ions) in the product of less than about 0.45 ppm, more preferably less than about 0.35 ppm, more preferably from 0.25 to about 0.35 ppm based on the weight of the composition product, e.g. the clay product.

In particular, it has been noted that improved anti-bacterial activity is observed when the composites of the present invention containing a silver metal and a titanium dioxide carrier are combined with an absorbing material which is a clay.

Any composition of the invention may further comprise a fragrance.

In a further aspect, the present invention provides a process for preparing a composition of the present invention comprising applying a liquid suspension of said biocidal composite as described above to an absorbent material as described above.

Such a liquid suspension can be prepared by suspending the composite in a solution of alcohol and sodium dioctyl sulphosuccinate salt (SDOSS). The SDOSS functions
as a suspension aid. A suitable concentration of SDOSS in such a liquid suspension is 15% by weight. A suitable concentration of composite in such a liquid suspension is 1 to 10% by weight.

An alternative described method of combining the composite and the absorbent material involves mechanically mixing the two components in water, extruding the resulting 10 product, and drying. Preferably, the final step involves drying to about 0-20% by weight of water.

In any method described above, the product may also be heat treated and calcined before or after the addition of the composite.

In a further aspect, the present invention provides the use of a biocidal composite as defined in any one of claims 1-2 to reduce bacteria in an absorbent material, wherein the absorbent material is part of an animal litter product.

The composites of the present invention can be employed to reduce levels of many microbes. An extensive list of such microbes against which silver/titanium dioxides composites are effective is provided in Corbett, R.J., International Journal of Cosmetic Science 18, 151-165 (1996).

In particular, the composites of the present invention are effective in reducing levels of the following bacteria: *Staphylococcus aureus, Staphylococcus epidermidis, Lactobacillus buchneri, Pseudomonas aeruginosa, Escherichia coli, Klebsiella pneumoniae, Seratia marcescens, Listeria monocytogenes, Bacillus subtilis,* and *Bacillus cereus.*

Also described is the use of a biocidal composite for reducing malodours in animal litter and/or animal bedding comprising an absorbent material,
wherein the biocidal composite comprises a transition metal compound and a carrier.

### Examples

Various compositions according to the invention were prepared and investigated to determine anti-bacterial/antimicrobial activity in comparison to compositions containing alternative anti-bacterial agents or no anti-bacterial agents.

Samples of commercially available cat litters (SANDY Traditional, and SANDY Clumping, both available from Geohellas) were treated with solutions of biocidal composites according to the invention, and tested.

For comparison purposes, two commercial cat litters marketed as anti-bacterial were also tested (EverClean Heavy Duty and EverClean with Activebloc).

### Antimicrobial Activity Testing

The compositions were tested against two indicator bacteria: *E. coli* and MRSA (Multiple-Resistant Staphylococcus Aureus).

Samples of the clays employed in the tested compositions were obtained, and the moisture holding capacity (MHC) of each material was determined using the Keens Raczkowski method (zero suction MHC) prior to testing for antimicrobial activity.

Replicate sub-samples (5g) of each tested composition were inoculated with aliquots of a suspension of either E. coli (2.3 x 10⁵ CFU ml⁻¹) or MRSA (2.2 x 10⁵ CFU ml⁻¹) in physiological saline solution to achieve 90% of the moisture holding capacity of te individual materials. The inoculated samples were then incubated at 35°C for up to 72 hours. After 24 and 72 hours incubation, replicate sub-samples (1g) were removed from each inoculated sub-sample and then suspended in an aliquot (9ml) of a neutriliser solution (SCDLP) and analysed for the presence of viable microorganisms by dilution plate count.

### Anti-Bacterial Additives

Two commercial silver chloride/titanium dioxide composites were obtained from ADDMASTER (BIOMASTER AT100 and TD100). AT100 is supplied in suspension form while TD100 is supplied as solid crystals. These products were sprayed on the absorbent materials (TD100 after dissolution in water) at the levels indicated below. AT100 is a liquid suspension prepared by suspending the composite in a solution of alcohol and sodium dioctyl sulphosuccinate salt.

Details of the compositions and the results of the antimicrobial testing are provided below.

### Examples 1-5

Examples 1 and 2 describe compositions comprising a clay absorbent material and a silver/titanium dioxide composite. Example 3 (comparison) describes a composition consisting only of the same clay absorbent material (without any antibacterial agent). Examples 4 and 5 (comparison) relate to commercially available cat litters which are marketed as anti-bacterial. Table 1 details the components of each sample tested.

**Table 1: Compositions of Examples 1-5**

| **Example** | **Clay Type** | **Anti-bacterial agent** | **Amount of agent % based on weight of clay.** |
|---|---|---|---|
| 1 | SANDY | TD100 | 0.012 |
| 2 | SANDY | AT100 | 1.7 |
| 3 | SANDY | --- | 0 |
| 4 | EverClean Heavy Duty | --- | --- |
| 5 | EverClean with Activebloc | --- | --- |

The antimicrobial test results relating to Examples 1-5 carried out as described above are provided in Table 2.

**Table 2: Antimicrobial Test Results Examples 1-5: Percentage Reduction in Colony Forming Bacteria from Initial**

| **Example** | **Composition** | **MRSA** | | ***Coli*** | |
|---|---|---|---|---|---|
| | | **24h** | **72h** | **24h** | **72h** |
| 1 | SANDY + TD100 | >99.89 | >99.89 | >99.88 | >99.88 |
| 2 | SANDY + AT100 | >99.88 | >99.88 | 99.87 | >99.88 |
| 3 | SANDY | --- | 82.21 | --- | --- |
| 4 | EverClean Heavy Duty | 84.35 | 93.00 | --- | --- |
| 5 | EverClean with Activebloc | 79.45 | 92.53 | --- | --- |

| | | | | | |
|---|---|---|---|---|---|
| No data indicates bacterial population growth. | | | | | |

It can be seen from the results that a strong antimicrobial effect is demonstrated by Examples 1 and 2 against MRSA after both 24 hours and 72 hours, whereby the populations were reduced to below the limit of detection. It is also seen from Examples 1 and 2 that a strong antimicrobial effect is demonstrated against *E*. *Coli .* After 72 hours, the populations of *E. Coli* were reduced to below the limit of detection.

Example 3 (without biocide) exhibits no antimicrobial effect whatsoever against *E*. *Coli,* and exhibits only moderate antimicrobial activity against MRSA after 72 hours, having shown an increase in microbial activity after 24 hours.

Examples 4 and 5 show that the commercially available cat litters marketed as antibacterial demonstrate no antimicrobial activity against *E. Coli,* and only a partial reduction in bacterial growth is achieved in relation to MRSA.

### Examples 6-15

Examples 6 to 15 describe further compositions according to the invention comprising a clay absorbent material and a silver/titanium dioxide biocidal composite. Examples 6-12 demonstrate the low levels of transition metal at which the compositions of the invention exhibit effective antimicrobial activity. Table 3 details the components of Examples 6 to 15.

**Table 3: Compositions of Examples 6-15**

| **Example** | **Clay Type** | **Anti-Bacterial Agent** | **Amount of Biocide % (based on weight of clay).** |
|---|---|---|---|
| 6 | SANDY Traditional | TD100 | 0.012 |
| 7 | SANDY Traditional | TD100 | 0.0036 |
| 8 | SANDY Traditional | TD100 | 0.006 |
| 9 | SANDY Traditional | AT100 | 0.17 |
| 10 | SANDY Traditional | AT100 | 0.51 |
| 11 | SANDY Traditional | AT100 | 0.85 |
| 12 | SANDY Clumping | TD100 | 0.0036 |
| 13 | SANDY Clumping | TD100 | 0.012 |
| 14 | SANDY Clumping | AT100 | 0.51 |
| 15 | SANDY Clumping | AT100 | 1.7 |

It can be seen from the results that a strong antimicrobial effect is demonstrated by Examples 6 to 15 against both *E*. *Coli* and MRSA after 24 hours, whereby the populations were reduced to below the limit of detection.

### Examples 16-17

Examples 16 and 17 relate to two samples reported in Table 1 above (Examples 1 and 2 respectively) that have been aged for 180 days in order to investigate any change in effectiveness over time.

## Claims

1. An animal litter product comprising a composition comprising (i) an absorbent material which is a clay and (ii) a biocidal composite which comprises an anti-bacterial transition metal compound and a carrier, wherein the transition metal compound is supported on the carrier material; and wherein the carrier material is titanium dioxide; and wherein the transition metal compound is present in the composition in an amount of less than 0.60ppm ; and wherein the transition metal compound is silver chloride.

2. The animal litter product of claim 1, wherein the clay is selected from the group consisting of sepiolite, zeolites, saponite, tobermite, marls, attapulgite, bentonite, kaolinite, halloysite, montmorillonite, smectite, vermiculite, hectorite, diatomaceous earth, Fuller's earth, synthetic cement, and mixtures thereof, preferably the clay is attapulgite, saponite, or mixtures thereof.

3. A process for preparing a composition as defined in any one of claims 1-2, comprising applying a liquid suspension of said biocidal composite to said absorbent material.

4. The process of claim 3, wherein the liquid suspension of said biocidal composite is applied to said absorbent material by spraying.

5. The use of a biocidal composite as defined in any one of claims 1-2 to reduce bacteria in an absorbent material, wherein the absorbent material is part of an animal litter product.

## Patentansprüche

1. Tierstreuprodukt, umfassend eine Zusammensetzung, umfassend (i) ein Absorptionsmaterial, das ein Ton ist, und (ii) einen bioziden Verbundstoff, der eine antibakterielle Übergangsmetallverbindung und einen Träger umfasst, wobei die Übergangsmetallverbindung auf dem Trägermaterial getragen wird; und wobei das Trägermaterial Titandioxid ist; und wobei die Übergangsmetallverbindung in der Zusammensetzung in einer Menge von weniger als 0,60 ppm vorliegt; und wobei die Übergangsmetallverbindung Silberchlorid ist.

2. Tierstreuprodukt nach Anspruch 1, wobei der Ton ausgewählt ist aus der Gruppe bestehend aus Sepiolith, Zeolithen, Saponit, Tobermit, Mergeln, Attapulgit, Bentonit, Kaolinit, Halloysit, Montmorillonit, Smektit, Vermiculit, Hectorit, Diatomeenerde, Fuller-Erde, synthetischem Zement und Mischungen davon, wobei der Ton vorzugsweise Attapulgit, Saponit oder Mischungen davon ist.

3. Verfahren zur Herstellung einer Zusammensetzung nach einem der Ansprüche 1 bis 2, umfassend ein Aufbringen einer flüssigen Suspension des bioziden Verbundstoffs auf das Absorptionsmaterial.

4. Verfahren nach Anspruch 3, wobei die flüssige Suspension des bioziden Verbundstoffs auf das Absorptionsmaterial durch Sprühen aufgebracht wird.

5. Verwendung eines bioziden Verbundstoffs nach einem der Ansprüche 1 bis 2 zur Verringerung von Bakterien in einem Absorptionsmaterial, wobei das Absorptionsmaterial ein Teil eines Tierstreuprodukts ist.

## Revendications

1. Produit de litière animale comprenant une composition comprenant (i) un matériau absorbant qui est une argile et (ii) un composite biocide qui comprend un composé de métal de transition antibactérien et un véhicule, où le composé de métal de transition est supporté sur le matériau véhicule ; et où le matériau véhicule est le dioxyde de titane ; et où le composé de métal de transition est présent dans la composition selon une quantité inférieure à 0,60 ppm ; et où le composé de métal de transition est le chlorure d'argent.

2. Produit de litière animale selon la revendication 1, dans lequel l'argile est choisie dans le groupe constitué par la sépiolite, les zéolithes, la saponite, la tobermorite, l'argile marneuse, l'attapulgite, la bentonite, la kaolinite, l'halloysite, la montmorillonite, la smectite, la vermiculite, l'hectorite, la terre de diatomées, la terre à foulon, le ciment synthétique, et des mélanges de ceux-ci, préférablement l'argile est l'attapulgite, la saponite, ou des mélanges de celles-ci.

3. Procédé de préparation d'une composition telle que définie selon l'une quelconque des revendications 1-2, comprenant l'application d'une suspension liquide dudit composite biocide audit matériau absorbant.

4. Procédé selon la revendication 3, dans lequel la suspension liquide dudit composite biocide est appliquée audit matériau absorbant par pulvérisation.

5. Utilisation d'un composite biocide tel que défini selon l'une quelconque des revendications 1-2, afin de réduire les bactéries dans un matériau absorbant, où le matériau absorbant fait partie d'un produit de litière animale.
